Europäisches Patentamt

European Patent Office

Office européen des brevets

(19)

(11) Numéro de publication : **0 078 225 B1**

(12)

# FASCICULE DE BREVET EUROPEEN

(45) Date de publication du fascicule du brevet :
**08.01.86**

(51) Int. Cl.⁴ : **G 01 N 33/52, B 01 L 3/14**

(21) Numéro de dépôt : **82401976.4**

(22) Date de dépôt : **26.10.82**

(54) **Procédé de mise en oeuvre d'analyses en chimie-biologique ainsi que dispositif et installation permettant la mise en oeuvre de ce procédé.**

(30) Priorité : **27.10.81 FR 8120179**

(43) Date de publication de la demande :
**04.05.83 Bulletin 83/18**

(45) Mention de la délivrance du brevet :
**08.01.86 Bulletin 86/2**

(84) Etats contractants désignés :
**AT BE CH DE GB IT LI LU NL SE**

(56) Documents cités :
**EP - A - 0 017 414**
**DE - A - 2 435 302**
**DE - A - 2 857 197**
**FR - A - 2 169 639**

(73) Titulaire : **Henry, Robert, 15, rue de Longjumeau, Ballainvilliers Essonne (FR)**

(72) Inventeur : **Henry, Robert, 15, rue de Longjumeau, Ballainvilliers Essonne (FR)**

(74) Mandataire : **Cabinet BERT, DE KERAVENANT & HERRBURGER, 115, Boulevard Haussmann, F-75008 Paris (FR)**

ACTORUM AG

**Description**

La présente invention a pour objet un procédé de mise en oeuvre d'analyses en chimie-biologique ainsi qu'un dispositif et un appareillage permettant la mise en oeuvre de ce procédé.

Depuis longtemps, on a cherché à effectuer de msnière automatique, diverses analyses habituelles en chimie biologique, et par suite, un certain nombre de procédés ainsi que de dispositifs ont déjà été proposés sur le marché.

Ces dispositifs et appareillages sont basés sur des technologies différentes, parmi lesquelles on peut noter le transfert des liquides par pompes péristaltiques (Technicon® ), ou le transfert des liquides par la force centrifuge (Centrifichem® , Cobas).

Depuis quelques temps, on a proposé des systèmes séquentiels, c'est-à-dire réalisant automatiquement plusieurs étapes de l'analyse qui utilisent un dispositif jetable à dose unitaire des réactifs. Ces appareillages, de par leur côté pratique et leur faible coût de revient, sont appelés, dans l'avenir , à connaître un développement important et attirent de plus en plus l'intérêt des techniciens.

Parmi les exemples de tels types d'appareillage qui ont déjà été proposés, on peut noter le système ACA® de DUPONT DE NEMOURS qui utilise des cassettes de réactifs constituées d'une enveloppe de plastique souple accrochée à une barrette rigide. Dans ce dispositif, la barrette supporte un code et l'enveloppe plastique est équipée de plusieurs cavités qui contiennent des réactifs en solution ou sous forme de comprimés, et une cavité qui sert au mélange de l'échantillon et des réactifs.

Par ailleurs, il a également été mis au point une installation permettant l'utilisation de ces cassettes de manière totalement automatique.

Dans le même ordre d'idée, on peut mentionner également le dispositif Ektachem® de KODAK qui utilise des films de 30 × 40 mm environ, formés de plusieurs couches de papier: certaines couches renferment les réactifs nécessaires, la couche supérieure est destinée à assurer la bonne répartition de l'échantillon déposé à la surface et la couche inférieure sert à réfléchir le faisceau lumineux utilisé pour le dosage par réflexion à travers les couches semi-transparentes.

Cependant, les dispositifs et appareillages mentionnés ci-dessus ne sont pas sans présenter un certain nombre d'inconvénients, étant donné que l'utilisation de réactifs sous forme de doses unitaires se heurte à la difficulté de conservation de certains des réactifs utilisés en chimie-biologique.

En effet, certains de ces réactifs d'origine biologique (principalement les enzymes et les anticorps) doivent être lyophilisés ou mis sous forme de comprimés, ce qui augmente notablement la difficulté de fabrication et la longueur nécessaire à la mise en oeuvre des analyses.

On a déjà proposé des procédés, dispositifs et appareillages permettant d'utiliser les avantages des solutions mentionnés ci-dessus, tout en remédiant à ces inconvénients, c'est-à-dire l'utilisation de «cassettes» renfermant des doses unitaires de réactifs spécifiques aux tests, dans lesquelles il ne reste plus qu'à ajouter l'échantillon à doser et un diluant, la cassette contenant la dose de réactifs étant ensuite purement et simplement jetée.

A titre d'exemple, on peut citer le brevet DE-A-2 435 302 qui décrit un procédé de mise en oeuvre d'analyses en chimie-biologique par lequel on utilise des doses unitaires de réactifs, et par lequel l'on absorbe une dose unitaire de réactifs biologiques liquides ou en solution sur un support solide constitué par une substance porteuse, notamment du papier en ajoutant un volume connu d'une solution aqueuse de ces réactifs à une concentration connue, on la sèche et on la désorbe de son support au moment de son utilisation par action d'un solvant aqueux approprié, l'évaporation de l'eau contenue dans la solution absorbée pouvant s'effectuer à l'air libre, sous air déshydraté ou dans un lyophilisateur.

Il s'agit là d'un procédé original, étant donné qu'il comporte une étape de désorption du réactif biologique de son support, per action d'un solvant aqueux approprié.

Il est reconnu qu'une alternative à la conservation par lyophilisation des produits d'origine biologique est l'introduction de ce produit dans une matrice poreuse et rigide qui lui sert de protection.

La présente invention a pour point de départ cette propriété qu'ont les substances poreuses, et plus particulièrement certains types de papier, de maintenir intactes les propriétés des substances biologiques qui y ont été absorbées en solution puis séchées.

Le matériau absorbant peut être du papier et plus particulièrement les papiers tels que ceux Réf. 2992 de Schleider et Schull et Réf. 818 de Macherey-Nagel, du coton sous différentes formes absorbantes, ou également des fibres synthétiques sous des formes absorbantes. L'objectif est d'obtenir une matrice semi-rigide poreuse qui protègera les substances biologiques.

Dans tous les cas, la stabilité est assurée selon des conditions de séchage qui peuvent varier d'une substance à l'autre mais qui nécessitent toujours une déshydratation assez complète.

La présente invention se propose d'améliorer les procédés décrits ci-dessus, en limitant l'absorption du ou des réactifs sur le support solide de façon telle que la désorption des réactifs soit plus facile et plus rapide et ce, tout en ajoutant aux réactifs, un composé qui puisse toujours être utilisé en chimie-biologique, ce qui exclut les tensio-actifs qui sont incompatibles avec certaines réactions telles que les réactions antigène/anticorps.

A cet effet, l'invention a pour objet un procédé de mise en oeuvre d'analyses en chimie-biologique par lequel on ultilise des doses unitaires de réactifs, et par lequel on absorbe une dose unitaire de réactifs biologiques liquides ou en solution sur un support solide constitué par une substance poreuse, notamment du papier en ajoutant un volume connue d'une solution aqueuse de ces réactifs à une concentration connue, on la sèche et on la désorbe de son support au moment de son utilisation par action d'un solvant aqueux approprié, l'évaporation de l'eau contenue dans la solution absorbée pouvant s'effectuer à l'air libre, sous air déshydraté ou dans un lyophilisateur, procédé caractérisé en ce que l'on ajoute aux réactifs préalablement a leur absorption, un agent susceptible d'accélérer la désorption constitué par une protéine et notamment de la sérum-albumine bovine. Par exemple, dans le cas des enzymes et des anticorps, on ajoute une quantité de sérum albumine bovine égale à 5 à 20 fois la quantité pondérale du réactif.

Par ailleurs, et selon une autre caractéristique de l'invention, l'étape de désorption se compose en fait de deux étapes qui peuvent être dans certains cas, quasi-simultanées.

En effet, on réhydrate tout d'abord le support solide par une solution aqueuse et quelque fois tamponnée, telle que par exemple le tampon phosphate pH 7,4, ensuite, on sépare de ce support solide la solution aqueuse imprégnante. On arrive ainsi à extraire le réactif de son support avec un volume extrêmement faible de l'ordre de 150 à 350 microlitres.

L'invention est donc basée sur la possibilité de disposer, sous une forme sèche, de réactifs qui peuvent être reconstitués facilement avec un volume minimum de solution, et ceci grâce à l'addition de protéines avant le séchage des réactifs.

Les modalités de mise en oeuvre du procédé décrit ci-dessus, ainsi que les dipositifs tels que les cassettes utilisées, peuvent être de plusieurs types, permettant une mise en oeuvre plus ou moins automatisée, sans pour cela sortir du cadre de l'invention.

Notamment, on peut déposer la dose unitaire de réactif(s) sur un ou plusieurs support(s) constitué(s) par un disque de papier que l'on laisse sécher à l'air, puis on place sous atmosphère sèche ou les disque(s) de papier dans un tube similaire au tube en plastique couramment utilisé en biologie que l'on ferme et ouvre pour y insérer la substance à analyser et effectuer la désorption puis on aspire la solution contenue dans le tube pour la transférer dans un appareillage d'analyse biologique connu en lui-même, notamment un photomètre à cellule de lecture fixe.

Il s'agit là en fait du processus d'application le plus simple du procédé qui est à la base de la présente demande; ce procédé particulièrement simple est bien adapté aux dosages des analyses non enzymes telles que l'urée, le glucose, le cholestérol, l'acide urique, etc.

Dans le cas où le test nécessite l'addition de deux réactifs, non pas simultanément, mais à des temps différents, il est possible que le second réactif soit déposé hors du tube, sur une feuille de papier mise dans une enveloppe plastique souple et étanche. Ces doses unitaires sont alors empilées en paquets et disposées dans un magasin alimentant, par un système à tiroir, un système perforant qui découpe des disques au diamètre voulu dans le papier et les enveloppes. Le mandrin perforant pousse ensuite les disques dans le tube situé en-dessous.

La mesure optique ne s'effectue bien entendu pas dans le tube à cause de la présence des disques de papier sur lesquels ont été déposés les réactifs sur un support venant se placer à l'extrémité supérieure d'un tube d'analyse biologique de type classique, on ajoute dans le tube la substance à analyser et sur ce ou ces disque(s) de papier les liquides nécessaires à la mise en oeuvre de l'étape de désorption, puis on pousse les liquides réactionnels à l'intérieur du tube en exerçant une pression d'air sur son sommet, et on effectue l'analyse notamment à l'aide d'un appareillage de mesure optique.

Lors de la mise en oeuvre de ce procédé, il est nécessaire que la décroissance de la pression soit réalisée lentement afin d'éviter que les disques ne soient projetés au-dessus du tube.

Selon l'invention, et dans certains cas particuliers, pour lesquels la substance à analyser est constituée pasr une substance pouvant elle aussi être absorbée et désorbée facilement, telle que par exemple le sang, on peut également utiliser le processus ci-dessus en absorbant en plus cette substance à analyser sur un support solide tel qu'un disque de papier similaire au support utilisé pour le ou les réactif(s). Dans ce cas, la désorption des substances à analyser s'effectue d'une manière identiques à la désorption des doses unitaires de réactifs.

La présente invention se rapporte également à un dispositif permettant la mise en oeuvre de procédé décrit ci-dessus, se composant d'un tube d'analyse de type classique ainsi que d'un support venant se placer à son extrémité supérieure.

Cependant, dans le cas où pour réaliser l'analyse, au moins deux réactifs doivent être ajoutés à des temps différents, il devient impossible d'utiliser des tubes d'analyse de type classique, tels qu'ils ont été décrits ci-dessus.

A cet effet, la présente invention propose un dispositif permettant la mise en oeuvre du procédé décrit ci-dessus, dans le cas où plusieurs réactifs doivent être ajoutés à des temps differents.

Ce dispositif est caractérisé en ce qu'il se compose d'une cassette en forme de parallélépipède creux en matériau plastique dont l'un des petites côtés est ouvert et destiné à recevoir un bouchon supportant au moins deux cônes en plastique ouverts à leurs extrémités équipés chacun d'un cône en papier filtre destiné chacun à recevoir une solution de réactifs, le bouchon comportant en outre, un trou destiné à permettre l'introduction des substances à analyser.

Les cônes en plastique sont en fait identiques pour leur forme à ceux utilisés dans les pipettes à embout jetable. Bien entendu, ils seront prévus en nombre correspondant au nombre de réactifs à utiliser.

Dans le cas où, comme mentionné précédémment, la substance à analyser est elle aussi absorbée sur un support solide similaire à celui où sont absorbés les réactifs, le trou destiné à permettre l'introduction des substances à analyser peut être supprimé et un cône en plastique supplémentaire est prévu.

La présente invention se rapporte également à une installation permettant, notamment à partir du dispositif décrit ci-dessus, la mise en oeuvre de façon automatique du procédé objet de l'invention.

Cette installation est caractérisée en ce qu'elle se compose d'un poste d'échantillonnage, équipé d'un diluteur dans lequel on prélève la substance à analyser et on l'introduit avec un volume donné de diluant dans la cassette, un poste d'analyse dans lequel on effectue l'introduction de la cassette ainsi que de données sur le patient, et le dosage à effectuer dans l'appareillage d'analyses biologiques, ainsi qu'un magasin de stockage des cassettes.

Le poste d'échantillonnage, ainsi que le magasin de stockage des cassettes, sont en fait des dispositifs qui sont connus en eux-mêmes, et ne nécessitent donc pas une description plus précise dans le cadre de cet exposé.

Le diluiteur peut être un produit commercial actuellement disponible; il en existe beaucoup de modèles dont certains sont très satisfaisants. Le magasin de cassettes, s'il doit être construit aux dimensions des cassettes ne comporte aucune innovation particulière. Il suffit simplement que l'accès à tous les types différents de cassettes soit facile et rapide. Un tourniquet portant un nombre de magasins verticaux correspondant au nombre de cassettes différentes, avec prélèvement manuel, simple par glissement au bas du magasin, donne toute satisfaction.

Selon une autre caractéristique de l'invention, le poste d'analyse comporte deux disques dont l'un est fixe et dont l'autre est mobile selon un mouvement pas-à-pas et comporte à sa périphérie des orifices destinés à recevoir les cassettes d'analyse; les cassettes sont maintenues par la table tournante, mais glissent sur la table fixe, et les deux disques sont situés à une certaine distance l'un de l'autre pour permettre l'insertion de l'appareillage d'analyse biologique, notamment des conduits de lumière du dispositifs de mesure optique.

Les dispositifs permettant la mise en oeuvre du procédé selon l'invention, sont décrits plus en détails en se référant aux dessins annexés qui représentent deux variantes de ce dispositif.

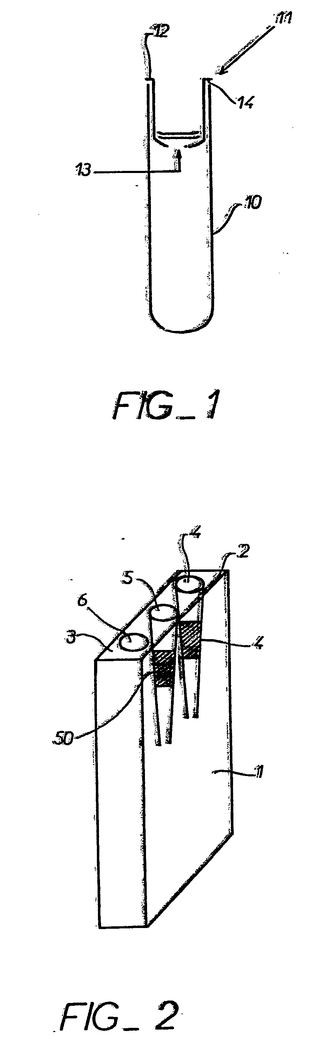

Selon la fig. 1, un dispositif particulièrement simple permettant la mise en oeuvre du procédé décrit ci-dessus est constitué par un tube d'analyses biologiques 10 de type classique à l'extrémité supérieure 11 duquel vient se placer un support annulaire 12 en un matériau quelconque, notamment en métal ou en un matériau synthétique. Ce support 12 présente à son extrémité inférieure un orifice 13 permettant le passage des liquides nécessaires à la mise en oeuvre de l'étape de désorption et à son extrémité supérieure un rebord 14 qui vient s'appuyer sur les extrémités supérieures 11 du tube 10 pour permettre de maintenir le support 12.

La fig. 2 se rapporte à un dispositif permettant d'effectuer des analyses biologiques dans lesquels au moins deux réactifs doivent être ajoutés à des temps différents.

Ce dispositif se compose d'une cassette 1 en forme de parallélépipède creux en matériau plastique dont l'un des petits côtés 2 est ouvert et reçoit un bouchon 3 qui supporte au moins deux cônes 4 et 5 équipés chacun de cônes en papier filtre 40 et 50 destinés chacun à recevoir une solution de réactifs. Le bouchon 3 comporte, en outre, un trou 6 destiné à permettre l'introduction des substances à analyser.

Les différentes variantes correspondant au procédé qui fait l'objet de l'invention, seront décrites plus en détail en se référant aux exemples ci-après.

### Exemple 1

Préparation et mise en oeuvre d'une analyse de glucose à l'aide du dispositif représenté sur la fig. 1.

Le réactif suivant a été préparé dans 5,6 ml d'eau distillée (méthode de Barthelmai et Crock):

| | | |
|---|---|---|
| - Hexokinase (source: levure) | 15 | UI |
| - Glucose-6-phosphate deshydro- | | |
| génase | 15 | UI |
| - (source: leuconostoe | | |
| mésentéroïdes) | | |
| - Adénosine triphosphate | 16,95 | mg |
| - Nicotinamide Adénine Dinucléotide | 7,5 | mg |
| - Chlorure de magnésium en tempon | | |
| triéthanol-amine | 51,3 | mg |
| - Chlorhydrate de triéthanolamine | 139 | mg |

2 × 150 microlitres du réactif décrit ci-dessus ont été déposée sur deux disques de 12 mm de diamètre en papier Réf. 2992 de Schleicher et Schull (U.S.A.). Ces disques ont été posés sur un support mince pour autoriser une circulation aussi complète que possible de l'air autour du papier.

Une lampe infrarouge a été dirigée sur les deux disques afin d'accélérer le séchage. Après quarante minutes, les deux papiers étaient secs.

Deux supports de diamètre extérieur 14 mm correspondant au dispositif représenté sur la fig. 1, ont été équipés avec les deux disques ci-dessus. Dans deux tubes, on a ajouté 5 microlitres d'un sérum à concentration connue de glucose dilué avec 50 microlitres d'eau distillée. Les deux supports précédemment décrits ont été disposés respectivement au-dessus des deux tubes, et 350 microlitres d'eau distillée ont été rajoutés sur chacun des deux supports.

Après une minute, une pression d'air a été exercée au-dessusu du support à l'aide d'une seringue et d'un bouchon.

Après six minutes de contact entre le sérum dilué et le réactif, une mesure a été effectuée sur un appareil Centrifichem 500 à 340 manomètres à 30°C.

On a ainsi lu deux valeurs très voisines, à savoir 0,75 et 0,77 g/litre inférieures à la valeur réelle du serum (0,88) car l'incubation a eu lieu à une température inférieure (27°C au lieu de 30°C).

### Exemple 2

Préparation et mise en oeuvre d'une analyse de glucose en utilisant un dispositif analogue à celui représenté sur la fig. 2.

On a utilisé un réactif correspondant à celui qui à été utilisé pour l'exemple 1.

150 microlitres de ce réactif ont été disposés sur un embout de pipette équipé d'un filtre Réf. 2992 de Schleicher et Schull. Ceci a été répété sur un deuxième embout.

Les deux embouts ont été mis à sécher en utilisant un jet d'air sec et chaud à 35°C dirigé sur le haut des embouts.

Le papier a ainsi séché en environ 30 minutes et à été conservé ainsi pendant 24 heures.

Afin de bien vérifier que l'élution de réactif donne un résultat convenable, 150 microlitres d'eau distillée ont été rajoutés au-dessusu de chacun des deux embouts et poussés à l'aide d'un pipette dans deux cuvettes d'un disque de transfert de Centrifichem 500, les deux positions «échantillon» de ces deux cuvettes ayant été préalablement remplies avec 5 microlitres (dilués avec 50 microlitres d'eau distillée) d'un sérum à concentration de glucose connue.

Après 6 minutes d'incubation à 30°C, le résultat a été lu à 340 nanomètres. On a trouvé des valeurs de 0,84 et 0,87 g/litres pour une valeur connue de 0,88 g/litre.

## Revendications

1. Procédé de mise en oeuvre d'analyses en chimie-biologique par lequel on utilise des doses unitaires de réactifs, et par lequel l'on absorbe une dose unitaire de réactifs biologiques liquides ou en solution sur un support solide constitué par une substance poreuse, notamment du papier en ajoutant un volume connue d'une solution aqueuse de ces réactifs à une concentration connue, on la sèche et on la désorbe de son support au moment de son utilisation par action d'un solvant aqueux approprié, l'evaporation de l'eau contenue dans la solution absorbée pouvant s'effectuer à l'air libre, sous air déshydraté ou dans un lyophilisateur, procédé caractérisé en ce que l'on ajoute aux réactifs préalablement à leur absorption, un agent susceptible d'accélérer la désorption constitué par une protéine et notamment de la sérum-albumine bovine.

2. Procédé selon la revendication 1, caractérisé en ce que l'on effectue la désorption en deux étapes, la première de ces étapes étant constituée par une réhydratation du support solide par une solution aqueuse, le cas échéant tamponnée, et la seconde par une séparation du support solide et de la solution des réactifs.

3. Dispositif permettant la mise en oeuvre du procédé selon l'une quelconque des revendications 1 et 2, dans le cas d'analyses biologiques dans lesquelles au moins deux réactifs doivent être ajoutées à des temps différents, caractérisé en ce qu'il se compose d'une cassette en forme de parallélépipède creux en matériau plastique dont l'un des petites côtés est ouvert et destiné à recevoir un bouchon supportant au moins deux cônes en plastique équipés chacun d'un cône en papier filtre destiné à chacun à recevoir une solution de réactifs, le bouchon comportant, en outre, un trou destiné à permettre l'introduction des substances à analyser.

## Patentansprüche

1. Verfahren zur Durchführung von biologischchemischen Analysen, bei welchem Einheitsdosen von Reaktionsmitteln verwendet werden und eine Einheitsdosis von flüssigen oder in Lösung befindlichen biologischen Reaktionsmitteln auf einem von einer porösen Substanz, insbesondere Papier, gebildeten festen Träger absorbiert wird, indem ein bekanntes Volumen einer wässrigen Lösung dieser Reaktionsmittel mit einer bekannten Konzentration zugegeben wird, diese getrocknet wird und im Zeitpunkt ihrer Verwendung durch Einwirkung eines geeigneten Lösungsmittels von ihrem Träger desorbiert wird, wobei die Verdampfung des in der absorbierten Lösung enthaltenen Wassers an freier Luft, unter entwässerter Luft oder in einem Gefriertrockner durchgeführt werden kann, dadurch gekennzeichnet, dass den Reaktionsmitteln vor ihrer Absorption ein Mittel zur Beschleunigung der Desorption beigegeben wird, das aus einem Protein und insbesondere aus Rinderserumalbumin besteht.

2. Verfahren nach Anspruch 1, dadurch gekennzeichnet, dass die Desorption in zwei Schritten durchgeführt wird, wobei der erste Schritt darin besteht, dass der feste Träger durch eine wässrige Lösung, ggf. abgepuffert, wiederbewässert wird, und der zweite darin, dass der feste Träger von der Lösung der Reaktionsmittel getrennt wird.

3. Vorrichtung zur Durchführung des Verfahrens nach einem der Ansprüche 1 und 2, für biologische Analysen, bei welchen wenigstens zwei Reaktionsmittel zu unterschiedlichen Zeitpunkten zugegeben werden müssen, dadurch gekennzeichnet, dass sie aus einer Kassette mit parallelepipedischer Hohlform aus Kunststoff besteht, deren eine kleine Seite offen und zur Aufnahme eines Stopfens bestimmt ist, der wenigstens zwei Kunststoffkegel trägt, deren jeder mit einem konischen Papierfilter ausgestattet ist, von denen jedes zur Aufnahme einer Reaktionsmittellösung bestimmt ist, wobei der Stopfen ausserdem ein Loch zur Einleitung der zu analysierenden Substanzen aufweist.

## Claims

1. A method of carrying out chemical-biological analyses according to which use is made of unit doses of reagents, and in conformity with which the absorption is effected of a unit dose of biological reagents, liquid or in solution, on a solid support constituted by a porous substance, particulary paper, by adding a known volume of an aqueous solution of these reagents in a known concentration, drying it and desorbing it from its support at the time of its use through the action of an appropriate aqueous solvent, it being possible for the evaporation of the water contained in the absorbed solution to be effected in the open air, in dehydrated air, or in a lyophilizer, a process characterised in that the addition is made to the reagents, prior to their absorption, of an agent able to accelerate desorption, constituted by a protein and particularly bovine serum-albumin.

2. A method according to Claim 1, characterised in that desorption is performed in two stages, the first of said stages consisting in a rehydration of the solid support by means of an aqueous solution, buffered if need be, and the second in a separation of the solid support and of the reagents solution.

3. An arrangement permitting the carrying out of the method according to either of Claims 1 and 2, in the case of biological analyses in which at least two reagents have to be added at different times, characterised in that it consists in a box in the shape of a hollow parallelepiped in synthetic plastic material, one of the small sides of which is open and intended to receive a plug supporting at least two cones of plastic material, each provided with a filter-paper cone, intended for each one to receive a solution of reagents, the plug comprising in addition a hole intended to permit the introduction of the substances to be analysed.

FIG_1

FIG_2